(19) Europäisches Patentamt
European Patent Office
Office européen des brevets

(11) **EP 1 643 538 B1**

(12) **EUROPÄISCHE PATENTSCHRIFT**

(45) Veröffentlichungstag und Bekanntmachung des Hinweises auf die Patenterteilung:
**17.06.2009 Patentblatt 2009/25**

(51) Int Cl.:
*H01J 65/04* (2006.01)    *F21V 11/12* (2006.01)
*F21V 17/00* (2006.01)    *A61L 9/00* (2006.01)

(21) Anmeldenummer: **05018819.2**

(22) Anmeldetag: **30.08.2005**

(54) **Dielektrisch behinderte Entladungslampe mit elektrischer Abschirmung**

Dielectric barrier discharge lamp with electrical screening

Lampe à décharge à barrière diélectrique avec blindage électrique

(84) Benannte Vertragsstaaten:
**AT DE FR GB HU**

(30) Priorität: **29.09.2004 DE 102004047374**

(43) Veröffentlichungstag der Anmeldung:
**05.04.2006 Patentblatt 2006/14**

(73) Patentinhaber: **Osram Gesellschaft mit beschränkter Haftung**
**81543 München (DE)**

(72) Erfinder:
- **Bschorer, Georg**
  **89542 Herbrechtingen (DE)**
- **Lecheler, Reinhard**
  **86633 Neuburg/Donau (DE)**
- **Lochschmidt, Andreas, Dr.**
  **89343 Jettingen-Scheppach (DE)**

(56) Entgegenhaltungen:
| | |
|---|---|
| EP-A- 0 517 929 | EP-A- 0 541 413 |
| EP-A- 0 860 655 | EP-A- 1 329 944 |
| WO-A-02/27762 | WO-A-97/40519 |
| WO-A-99/48134 | DE-A1- 10 111 447 |
| DE-A1- 10 213 195 | JP-A- 2001 319 510 |
| US-A1- 2003 024 804 | |

EP 1 643 538 B1

## Beschreibung

### Technisches Gebiet

Die vorliegende Erfindung bezieht sich auf eine dielektrisch behinderte Entladungslampe. Darunter versteht man Entladungslampen, bei denen zumindest die Anoden oder bei bipolarem Betrieb auch sämtliche Elektroden durch eine dielektrische Schicht von einem Entladungsmedium in dem Entladungsgefäß getrennt sind. Dadurch kommt es in Folge einer elektrischen Aufladung der dielektrischen Schicht auf der Anode bzw. der in dieser Phase als Anode wirkenden Elektrode zu einem eigenständigen Erlöschen der Entladung durch eine innere Gegenpolarisation. Der Lampenbetrieb erfolgt also letztlich durch eine dichte Reihe sehr kurzer Entladungsblitze.

### Stand der Technik

Solche dielektrisch behinderte Entladungslampen sind in verschiedener Weise im Stand der Technik bekannt geworden und aufgrund verschiedener vorteilhafter technischer Eigenschaften insbesondere für die Hinterleuchtung von Anzeigevorrichtungen, etwa Computermonitoren und Fernsehbildschirmen, oder für Büroautomationsanwendungen von Interesse. Im letztgenannten Fall werden i. d. R. langgestreckt stabförmige Lampenformen eingesetzt, die zur Beleuchtung von Dokumenten in Scannern, Faxgeräten, Kopierern und dgl. dienen können. Solche Entladungslampen mit einem röhrenförmig langgestreckten Entladungsgefäß sind ebenfalls bereits bekannt und erhältlich. Sie können auch für andere Anwendungen, beispielsweise als UV-Strahler für bestimmte technische Prozesse, von Interesse sein. Die vorliegende Erfindung ist nicht auf einen bestimmten Anwendungsfall eingeschränkt.

Dielektrisch behinderte Entladungslampen können aufgrund des kurz umrissenen Entladungsmechanismus nicht mit Gleichstrom betrieben werden, sondern werden entweder mit unipolaren Leistungsversorgungspulsen oder mit bipolaren Leistungsversorgungspulsen betrieben. Die verwendeten Frequenzen liegen im Regelfall in der Größenordnung einiger 10 kHz, so dass solche Entladungslampen in EMV-empfindlichen Umgebungen Störstrahlung erzeugen.

Es ist daher ebenfalls bereits bekannt, bei dielektrisch behinderten Entladungslampen mit röhrenförmig langgestreckten Entladungsgefäßen metallische Abschirmungen zu verwenden. Als Beispiel kann verwiesen werden auf die EP 0 981 831, auf die weiter unten noch Bezug genommen wird.

Die metallischen Abschirmungen müssen das Entladungsgefäß teilweise umgreifen und andererseits einen bestimmten Bereich zur Lichtabstrahlung freilassen. Da die Lichtabstrahlungsgeometrie i. d. R. entlang der Längserstreckung weitgehend gleich bleibt, wird hier von einem Öffnungswinkel gesprochen. Dieser Öffnungswinkel tritt also in einer Schnittebene senkrecht zur Längserstreckungsrichtung auf.

### Darstellung der Erfindung

Der Erfindung liegt das technische Problem zugrunde, eine dielektrisch behinderte Entladungslampe mit röhrenförmig langgestrecktem Entladungsgefäß und leitfähiger metallischer Abschirmung des Entladungsgefäßes anzugeben, die hinsichtlich der EMV-Verträglichkeit in vorteilhafter Weise weiterentwickelt ist. Die Erfindung soll ferner ein entsprechendes Beleuchtungssystem mit einer solchen Entladungslampe und einem zugehörigen elektronischen Vorschaltgerät angeben.

Dieses technische Problem wird gelöst durch eine dielektrisch behinderte Entladungslampe mit einem röhrenförmig langgestreckten Entladungsgefäß und mit einer leitfähigen metallischen Abschirmung, die das Entladungsgefäß teilweise umgreift und dabei in einer Schnittebene senkrecht zur Längserstreckungsrichtung des Entladungsgefäßes einen Öffnungswinkel zur Lichtabstrahlung freilässt, gekennzeichnet durch zumindest eine sich längs des Entladungsgefäßes erstreckende und den Öffnungswinkel begrenzende Schirmfläche der Abschirmung, die von dem Entladungsgefäß an ihrem äußersten Ende um eine Strecke entfernt ist, die mindestens so groß wie der halbe mittlere Durchmesser des Entladungsgefäßes quer zur Längserstreckung ist.

Ferner bezieht sich die Erfindung auch auf ein Beleuchtungssystem mit einer solchen Entladungslampe und einem elektronischen Vorschaltgerät zum Betrieb der Lampe, an dem die Entladungslampe elektrisch angeschlossen ist, wobei die elektrische Verbindung zwischen dem Vorschaltgerät und der Entladungslampe abgeschirmt ist.

Vorteilhafte Ausgestaltungen sind in den abhängigen Ansprüchen angegeben und werden im Folgenden näher erläutert. Es wird vorsorglich darauf hingewiesen, dass die dabei offenbarten Merkmale implizit auch als Offenbarung eines Verfahrens zum Montieren einer Entladungslampe und eines Beleuchtungssystems und als Betriebsverfahren für die Entladungslampe und das Beleuchtungssystem verstanden werden können.

Röhrenförmige Entladungslampen dieses Typs weisen entlang ihrer Längserstreckung eine sog. Apertur auf, also einen längs verlaufenden Streifen, aus dem Licht aus der Lampe austritt. Zur Gewährleistung einer guten Effizienz sollte diese Apertur möglichst nicht direkt durch eine Abschirmung abgedeckt werden, weswegen bekannte Abschirmungen die Apertur auch vollständig aussparen. Allerdings strahlt die Lampe dann über den gesamten ausgesparten Bereich in den entsprechenden Raumwinkel ab. Die durch die Erfindung vorgesehene Schirmfläche begrenzt den Raumwinkel dieser Abstrahlung und definiert damit auch einen Öffnungswinkel der Lichtabstrahlung. Dieser Öffnungswinkel kann auf die technisch gewünschte Anwendung hin optimiert sein, d. h. im Einzelfall kann der Öffnungswinkel auch deutlich kleiner sein als bei gegebener Apertur eigentlich möglich. In diesem Fall würde jedoch die Schirmfläche die Lichtausbeute in dem für die Anwendung relevanten Raumwinkel nicht beeinträchtigen, wohl aber die Abschirmung deutlich verbessern.

Die Grundidee der Erfindung besteht somit darin, dass die Abschirmung nicht auf eine an sich bekannte leitfähige Umhüllung des Entladungsgefäßes außerhalb des Öffnungswinkels begrenzt wird, sondern dass die Abschirmung zumindest eine Schirmfläche aufweist, die sich von dem Entladungsgefäß wegerstreckt und dabei den Öffnungswinkel begrenzt. Die Abschirmung soll also gewissermaßen eine "Blende" entlang zumindest einer seitlichen Grenze des Öffnungswinkels aufweisen. Vorzugsweise sind an beiden Grenzen des Öffnungswinkels entsprechende Schirmflächen vorgesehen, jedoch könnte eine Schirmfläche auch entfallen, beispielsweise wenn die Abschirmung in die andere Richtung nicht wesentlich ist oder aus anderen Gründen, etwa durch eine ohnehin dort vorhandene metallische Wand, schon gegeben ist. Die Schirmfläche muss dabei nicht notwendigerweise entlang ihrer gesamten Erstreckung entlang der Grenze des Öffnungswinkels laufen, also nicht notwendigerweise im Wesentlichen radial verlaufen. Vorzugsweise begrenzt zumindest ihr äußerstes Ende den Öffnungswinkel. Dieses äußerste Ende ist im Übrigen erfindungsgemäß zumindest um den halben mittleren Durchmesser des Entladungsgefäßes von dem Entladungsgefäß entfernt.

Es ist im Übrigen auch nicht unbedingt notwendig, dass die Abschirmung von dem Öffnungswinkel abgesehen den gesamten übrigen Umfang des Entladungsgefäßes umgibt. Auch hier können durch Bedeutungslosigkeit der EMI-Abstrahlung in eine bestimmte Richtung oder dort ohnehin vorgesehene abschirmende Elemente die Gründe für eine Abschirmung fehlen und/oder andere bauliche Gründe gegeben sein, die eine Lücke in der Abschirmung vorteilhaft erscheinen lassen.

Es ist allerdings im Rahmen dieser Erfindung bevorzugt, dass die Abschirmung das Entladungsgefäß um mehr als die Hälfte seines Umfangs umgreift und abschirmt und damit gewissermaßen eine Manschette bildet. Diese Manschette kann, wie im Folgenden noch näher ausgeführt, auch vorteilhafte Eigenschaften als Montagehilfe oder Halterung haben.

Die erwähnte Manschette hat vorzugsweise zu einem Teil des Umfangs des Entladungsgefäßes, besonders bevorzugter Weise zu dem, von der oder den Schirmflächen abgesehen, übrigen Teil, einen relativ kleinen Abstand von dem Entladungsgefäß, und zwar im Verhältnis zu dem mittleren halben Durchmesser des Entladungsgefäßes. Der übrigen Teil der Abschirmung bildet dann die erwähnte Schirmfläche. Zur Veranschaulichung wird auf die Ausführungsbeispiele verwiesen.

Zwar kann die erfindungsgemäße Schirmfläche der Abschirmung die Lichtabstrahlung der Lampe begrenzen und damit einen effektiven Öffnungswinkel zumindest zu einer Seite hin definieren. Andererseits ist in vielen Fällen erwünscht, einen möglichst großen Teil des abgestrahlten Lichts auszunutzen. Bezieht man die Erstreckung der Apertur auf den Mittelpunkt des Entladungsgefäßes im Querschnitt zur Längsrichtung und betrachtet dies als Öffnungswinkel, so sollte vorzugsweise der auf den gleichen Mittelpunkt bezogene Lichtabstrahlungsöffnungswinkel der Abschirmung größer als der der Apertur sein. Dabei kann übrigens die Schirmfläche durchaus noch von der Apertur abgestrahltes Licht abblenden, weil die Lichtabstrahlung in der Lampe auch von der Apertur näheren Teilen des Innenmantels her erfolgt, so dass der effektive Lichtabstrahlungswinkel der Apertur größer als der radial betrachtete Öffnungswinkel ist.

Ferner kann die Abschirmung neben der oder den Schirmflächen auch weitere abschirmende Elemente im Bereich des Öffnungswinkels enthalten, insbesondere im Querschnitt im Wesentlichen radial verlaufende flächige Abschirmungsteile, die den Öffnungswinkel weiter unterteilen. Damit kann die Abschirmung auch in Richtung der Lichtabstrahlung etwas verbessert werden. Beispiele werden weiter unten erläutert.

Es kann von Bedeutung sein, dass die Abschirmung nicht zu stark kapazitiv an eine oder eine Mehrzahl außenliegende Elektroden ankoppelt. Hierbei ist bevorzugt, dass eine angenommene radiale Dicke $d_D$ zwischen der metallischen Abschirmung und der außenliegenden Elektrode, also etwa die Dicke der erwähnten Isolationsschicht innerhalb der Metallabschirmung, und eine Dielektrizitätszahl $\varepsilon_D$ dieser Schicht sowie eine Dicke $d_B$ der dielektrischen Barriere zwischen der Elektrode und dem Entladungsmedium bei einer entsprechenden Dielektrizitätszahl $\varepsilon_B$ insgesamt die Beziehung erfüllen:

$$d_D / \varepsilon_D \geq F \times d_B / \varepsilon_B,$$

wobei der Faktor F zumindest 1,5, bevorzugt zumindest 2 und besonders bevorzugt zumindest 2,5 beträgt. Zu weiteren Einzelheiten wird verwiesen auf die EP 0 981 831, in der u. a. auch erläutert wird, dass in dieser Beziehung im Fall mehrschichtiger Aufbauten die entsprechende Summe der einzelnen Quotienten aus Dicke und Dielektrizitätszahl verwendet werden muss.

Eine einfache und bevorzugte Möglichkeit besteht darin, zumindest einen, vorzugsweise zwei endseitige Sockel an der Lampe vorzusehen, die radial etwas größer bemessen sind als das Entladungsgefäß selbst. Wenn dann die Abschirmung in anliegender Weise auf die Sockel aufgebracht und vorzugsweise auch in dieser Form montiert und gehalten wird, ist durch den radialen Unterschied zwischen Sockel und Entladungsgefäß der gewünschte Abstand gegeben.

Es kann dabei durchaus sinnvoll und bevorzugt sein, die Abschirmung das Entladungsgefäß teilweise berühren zu lassen. Zum einen kann die Abschirmung damit auch als Kühlkörper und im Hinblick auf eine Temperaturhomogenisierung entlang der Längserstreckung der Lampe wirken. Ferner kann so eine möglicherweise vorgesehene leitende Schicht auf dem Entladungsgefäß selbst leitend mit der Abschirmung verbunden werden, etwa eine Indium-Zinn-Oxidschicht, wie sie in der bereits zitierten EP 0 981 831 erwähnt ist. Eine solche leitende Schicht kann transparent sein, was im Fall des Indium-Zinn-Oxids der Fall ist. Dann kann sie über die Apertur erstreckt sein und damit auch in diesem Bereich eine gewisse Abschirmwirkung entfalten. Eine solche leitende transparente Schicht zur Abschirmung insbesondere im Bereich der Apertur ist natürlich auch unabhängig von einer durch direkte Berührung erfolgende Kontaktierung mit der Abschirmung im Sinn der vorliegenden Erfindung bevorzugt.

Eine weitere bevorzugte Ausgestaltung des Sockels betrifft Abflachungen an seiner Querschnittsform (senkrecht zur Längserstreckung des Entladungsgefäßes), die in passender Weise auch an der Abschirmung, etwa einem entsprechend geformten Metallblech, vorgesehen sind. Dann kann bei der Montage der Abschirmung an den Sockeln durch die Ausrichtung der Abflachungen eine korrekte Orientierung, also insbesondere eine Ausrichtung einer Apertur der Lampe auf den durch die Abschirmung definierten Öffnungswinkel, vorgegeben werden. Dabei kann der Sockel natürlich auch weitere Rastvorrichtungen enthalten, die zu der Abschirmung passen. Es kann jedoch auch alleine durch die Manschettenform, d. h. durch den Formschluss der Abschirmung selbst, eine Verrastung oder Klemmwirkung gegeben sein.

Die bereits erwähnte Schirmfläche bzw. die vorzugsweise vorgesehenen beiden Schirmflächen an den Seiten des Öffnungswinkels können neben einer Verbesserung der Abschirmwirkung auch andere Aufgaben erfüllen. Sie können insbesondere auch zu Montage- und Halterungszwecken verwendet werden.

Die Abschirmung kann nämlich ausgestaltet sein als eine Manschette, wobei zumindest eine äußere Elektrode an dem Entladungsgefäß durch Formschluss mit der die Elektrode umgreifenden Manschette angebracht ist, welche Manschette den Umfang des Entladungsgefäßes senkrecht zu der Längserstreckung teilweise umgreift, dabei jedoch eine Apertur zur Lichtabstrahlung freilässt.

Dies hat auch Bedeutung für ein entsprechendes Herstellverfahren, bei welchem zumindest eine Elektrode durch einen Formschluss mit der die Elektrode umgreifenden Manschette an einem röhrenförmig langgestreckten Entladungsgefäß so angebracht wird, dass die Elektrode entlang der Längserstreckung des Entladungsgefäßes liegt, wobei die Manschette eine Apertur zur Lichtabstrahlung freilässt.

Die Grundidee liegt dabei darin, zur Montage der zumindest einen Elektrode oder vorzugsweise der zwei oder auch mehreren außenliegenden Elektroden eine Manschette zu verwenden, die mit der Abschirmung einheitlich ist. Mit Manschette wird hier eine Vorrichtung bezeichnet, die eine eigene ausreichende Formstabilität hat, um die Elektroden durch Formschluss zu halten. Die Manschette soll also sozusagen als Klammer oder Klemmvorrichtung eingesetzt sein. Dies erlaubt, eine Apertur zur Lichtabstrahlung durch die Entladungslampe freizulassen, so dass die Manschette nicht besonders dünn und nicht transparent ausgeführt sein muss. Die Manschette muss ferner nicht aufgeklebt werden. Sie erlaubt darüber hinaus eine Stabilisierung und/oder einen Schutz des Entladungsgefäßes gegen äußere Einwirkungen und kann damit auch zu einer aus Gewichtsgründen und zur Vermeidung zu hoher Spannungen gewünschten Verringerung der Wandstärken des Entladungsgefäßes beitragen. Insbesondere lässt sich die Elektrode an dem Entladungsgefäß durch einfaches Aufklipsen oder Einschieben der bzw. in die Manschette montieren, so dass die Herstellung der Entladungslampe an dieser Stelle deutlich vereinfacht und beschleunigt wird.

Bevorzugt ist, dass nur der erwähnte Formschluss die Elektrode hält, also diese nicht noch darüber hinaus an dem Entladungsgefäß angeklebt oder in anderer Weise befestigt ist, und ferner, dass die Manschette zu diesem Zweck unter Vorspannung steht, also auch im montierten Zustand noch einen gewissen Anpressdruck aufrecht erhält.

Ferner ist auch bevorzugt, dass die Manschette selbst an dem Entladungsgefäß nur durch Formschluss oder auch Kraftschluss in Folge ihrer Eigenstabilität gehalten ist, also an sich frei anliegt. Sie soll also ebenfalls nicht zusätzlich angeklebt sein.

Vor allem im Hinblick auf die bereits erwähnte Stabilisierungs- und Schutzfunktion der Manschette ist es zwar bevorzugt, im Rahmen der Erfindung aber durchaus nicht notwendig, dass sich die Manschette im Wesentlichen entlang dem gesamten Entladungsgefäß erstreckt. Es können im Einzelfall auch eine oder eine Mehrzahl Manschetten Verwendung finden, die nur einen Teil der Längserstreckung des Entladungsgefäßes ausmachen.

Ferner ist die oben stehende Erläuterung zu dem Formschluss und der eigenen Formstabilität der Manschette nicht so zu verstehen, dass diese notwendigerweise einstückig sein muss. Es ist im Rahmen einer besonderen Ausgestaltung der Erfindung im Gegenteil vorgesehen, eine zumindest zweiteilige Manschette zu verwenden. Dabei kann auch eine Funktionsdifferenzierung stattfinden, etwa in Form der äußeren Abschirmung und einer darin liegenden elektrischen Isolierung zwischen zumindest der Elektrode und der Abschirmung. In solchen Fällen muss die Isolierung selbst nicht unbedingt formstabil sein, wenngleich sie als Teil der Manschette aufzufassen ist.

Eine weitere Möglichkeit für eine zwei- oder mehrteilige Manschette besteht in zwei entlang der Längserstreckung des Entladungsgefäßes geteilten und im montieren Zustand aneinander anschließenden und fest miteinander verbundenen Teilen, die im verbundenen Zustand gegenüber dem Entladungsgefäß einen Form- oder Kraftschluss herstellen. Solche Teile können also auch ohne Form- und Kraftschluss an das Entladungsgefäß angelegt und dann zur Herstellung des Form- oder Kraftschlusses miteinander verbunden werden. In Betracht kommen insbesondere Schraub- oder Klipsverbindungen zwischen den beiden Teilen, vorzugsweise auch unlösbare Klipsverbindungen. Diese Ausführungsform eignet sich besonders für Manschetten, die aus nicht wesentlich elastischem Material bestehen.

Bei einer weiteren Ausgestaltung sind die außenliegenden Elektroden stabförmig ausgebildet und werden an einem Ende als Steckverbindungselemente benutzt. Stabförmig bedeutet dabei, dass die Elektroden eine gewisse eigene Formstabilität aufweisen und damit als Steckverbindungselement benutzbar werden, also keine Folienelektroden sind. Insbesondere sollten dabei Länge und Breite der Elektroden quer zur Längserstreckung größenordnungsmäßig vergleichbar sein, beispielsweise um nicht mehr als einen Faktor 5 voneinander abweichen.

Die Elektroden sollen dabei so ausgestaltet sein, dass sie in einer mechanisch vorzugsweise lösbaren, d. h. ohne grundsätzliche Zerstörung wieder trennbaren, Form mit einem komplementären Steckverbindungselement verbunden werden können. Unter Steckverbindung wird dabei eine unter Beibehaltung der wesentlichen Form der Steckverbindungselemente erfolgende kraftschlüssige Verbindung von in sich weitgehend formstabilen Elementen verstanden. Damit soll die Steckverbindung abgegrenzt werden von beispielsweise Crimpverbindungen, bei denen folienartige Elektroden bei wesentlicher Änderung ihrer Form und ohne Ausnutzung einer Formstabilität kontaktiert werden.

Die Ausnutzung der Elektroden selbst als Steckverbindungselemente sorgt für einen einfachen Aufbau und vereinfacht das Kontaktierungsverfahren deutlich.

Insbesondere können die Elektroden einfache Rundstäbe sein und dabei entweder als sog. weibliches Element der Steckverbindung ein Rohrende aufweisen oder als sog. männliches Element als Rundstab enden. Das zur Aufnahme eines Rundstabes ausgebildete Rohrende als weibliches Steckverbindungselement kann also sowohl elektrodenseitig als auch kabel- oder vorschaltgerätseitig vorliegen. Entsprechende Ausgestaltungen sind natürlich auch mit anderen als runden Querschnitten möglich, wobei die runde Querschnittsform jedoch bevorzugt ist.

Die Erfindung bezieht sich übrigens auch auf solche Entladungslampen, bei denen die zumindest zwei Gegen-Steckverbindungselemente für die beschriebenen Elektrodenenden mit inbegriffen sind, die also beispielsweise bereits mit einem Kabel versehen oder zusammen damit verpackt sind. Bevorzugt ist dabei nicht nur eine zerstörungsfrei lösbare Steckverbindung, sondern darüber hinaus eine über rein translatorische Bewegung herstellbare Steckverbindung. Solche Steckverbindungen sind strukturell einfach und erlauben ein besonders einfaches Kontaktierungsverfahren.

Günstige geometrische Ausgestaltungen für die Steckverbindungselemente an den Elektroden oder die komplementären Steckverbindungselemente sind so gestaltet, dass ein Element das komplementäre zumindest teilweise umgreift. Beispielsweise wird bei der geschilderten Verbindung zwischen einem Stab- und einem Rohrende das Stabende von dem Rohrende vollständig umgriffen. Wenn jedoch ein verbreitertes Flachende eines Stabes in einen Schlitz eines komplementären Elements eingesteckt wird, so ist das Flachende nur noch an zwei Seiten, also nur teilweise, von dem komplementären Element umgriffen. Hier ist also gemeint, dass ein Element in Bezug auf die Längsrichtung "seitlich" auf zumindest zwei Seiten des anderen anliegt.

Vorzugsweise stehen die als Steckverbindungselemente zu nutzenden Elektrodenenden über die Entladungslampe über und sind damit zur Verbindung mit den komplementären Steckverbindungselementen besonders gut erreichbar. Eine weitere Ausgestaltung der Erfindung sieht eine modulare Reihung einzelner Entladungsgefäße vor, die quasi als einheitliche Entladungslampe gemeinsam betreibbar sind. Im Fall der bereits erwähnten Steckverbindungen am Ende stabförmiger Elektroden können die Elektroden der einzelnen Module zusammengesteckt werden und dabei könnten die Manschetten einzelner Module ebenfalls miteinander verbunden oder nur aneinander angrenzend ausgestaltet sein, es könnte jedoch auch eine durchgehende Manschette für eine Mehrzahl Module verwendet werden. Auch ohne die erwähnte Steckverbindung kann diese Ausgestaltung vorteilhaft sein, etwa wenn die Entladungsgefäße in der beschriebenen Weise modular aneinandergereiht sind und durch modulare oder durchgehende Manschetten gehalten sind und dabei durchgehende außenliegende Elektroden in der erfindungsgemäßen Weise durch die Manschette(n) gehalten sind.

Die Entladungslampe ist im Betrieb mit einem elektronischen Vorschaltgerät zu verbinden, das die bereits erwähnte gepulste Leistungsversorgung sicherstellt. Dabei ist die elektrische Verbindung zwischen der Lampe und dem Vorschaltgerät vorzugsweise ebenfalls abgeschirmt. Dies kann durch eine Abschirmung eines Kabels erfolgen, aber auch durch eine direkte räumliche Nähe zwischen der Entladungslampe und einem abschirmenden Gehäuse des Vorschaltgeräts, wobei beide leitend miteinander verbunden werden und die elektrische Verbindung damit mit abdecken.

Insbesondere kann dabei die Abschirmung auch Montagefunktion haben, also mit dem Vorschaltgerätgehäuse fest verbunden sein und die Entladungslampe halten.

Eine Ausgestaltung der Erfindung bezieht sich auf ein Beleuchtungssystem mit der dielektrisch behinderten Entladungslampe, bei dem mit einem Gehäuse des Vorschaltgeräts ein Steckverbindungselement fest verbunden ist, das so ausgelegt ist, dass die Lampe mit dem die Kontakte aufweisenden Ende als komplementäres Steckverbindungselement durch Zusammenstecken mit dem Steckverbindungselement des Gehäuses an das Vorschaltgerät angeschlossen werden kann.

Ferner bezieht sich dieser Aspekt auf ein Verfahren zum Anschließen der Entladungslampe an dem elektronischen Vorschaltgerät, bei welchem die Entladungslampe mit einem Kontakte zu einem elektrischen Anschluss der Lampe aufweisenden Ende als Steckverbindungselement in ein dazu komplementär ausgebildetes Steckverbindungselement eingesteckt wird, das mit dem Gehäuse des Vorschaltgeräts fest verbunden ist.

Die Grundidee besteht darin, eine dielektrisch behinderte Entladungslampe mit röhrenförmig langgestrecktem Entladungsgefäß gewissermaßen selbst als Steckverbindungselement aufzufassen. Dazu weist die Entladungslampe an einem Ende angebrachte Kontakte zum elektrischen Anschluss auf und wird mit diesem Ende mit einem entsprechend ausgestalteten komplementären Steckverbindungselement verbunden, das mit dem Vorschaltgerät, d. h. dessen Gehäuse fest verbunden ist. Natürlich kann dabei das vorschaltgerätseitige Steckverbindungselement über ein Kabel mit einer Schaltungsplatine des Vorschaltgeräts verbunden sein, jedoch soll durch die Steckverbindung eine direkte mechanische Verbindung zwischen Lampe und Vorschaltgerät geschaffen sein.

Bevorzugt ist dabei, dass das vorschaltgerätseitige Steckverbindungselement nicht nur mit dem Gehäuse fest verbunden, sondern in das Gehäuse integriert ist. In anderen Worten soll das Steckverbindungselement kein fester Anbau sein. Es soll also auf ein flexibles Kabel zwischen dem Vorschaltgerätgehäuse und der Lampe im Sinne einer flexiblen mechanischen Verbindung dazwischen verzichtet werden. Bevorzugt ist, dass das Steckverbindungselement in dem Vorschaltgerätgehäuse flächig integriert ist, also beispielsweise als Ausnehmung in einem im Übrigen z. B. quaderförmigen Gehäuse, in welche Ausnehmung die röhrenförmige Lampe selbst mit einem Ende eingesteckt werden kann. Zur Veranschaulichung wird auf das Ausführungsbeispiel verwiesen.

Das vorschaltgerätseitige Steckverbindungselement ist vorzugsweise eine Steckbuchse, also ein weibliches Element in Bezug auf die Röhrenform der Lampe.

Bevorzugte Anwendungen der erfindungsgemäßen Entladungslampe und des erfindungsgemäßen Beleuchtungssystems liegen nicht nur in der Büroautomation, sondern auch bei UV-Strahlern. Solche UV-Strahler können für verschiedene technische Prozesse verwendet werden. Von besonderem Interesse ist im Rahmen dieser Erfindung die Beleuchtung von Katalysatoroberflächen zur Fotokatalyse von Reaktionen. Ein bevorzugtes Beispiel für eine Anwendung liegt in der Luftreinigung, insbesondere in Fahrzeugen, etwa Kraftfahrzeugen. Hier können Luftschadstoffe durch einen fotokatalytischen Prozess umgewandelt und damit beseitigt werden und somit der Fahrzeuginnenraum mit einer gegenüber der Außenwelt qualitativ deutlich verbesserten Luft versorgt werden.

## Kurze Beschreibung der Zeichnungen

Im Folgenden wird die Erfindung anhand von Ausführungsbeispielen näher erläutert, wobei die einzelnen Merkmale auch in anderen Kombinationen erfindungswesentlich sein können.

| | |
|---|---|
| Figur 1 | zeigt eine schematische perspektivische Ansicht eines erfindungsgemäßen Beleuchtungssystems. |
| Figur 2 | zeigt das Beleuchtungssystem aus Figur 1 bei von dem Vorschaltgerät abgenommener Entladungslampe. |
| Figur 3 | zeigt eine schematische Draufsicht auf das Beleuchtungssystem aus Figur 1. |
| Figur 4a | zeigt eine schematische perspektivische Ansicht eines Endes der Entladungslampe aus den Figuren 1 - 3 gemäß einer alternativen Ausführungsform. |
| Figur 4b | zeigt eine Variante zu Figur 4a. |
| Figuren 5 - 9 | zeigen jeweils schematische Frontansichten von Entladungslampen nach alternativen Ausführungsformen. |
| Figur 10 | zeigt eine perspektivische Darstellung einer Variante eines Abschirmblechs der Entladungslampe aus den Figuren 1 - 3. |
| Figur 11 | zeigt eine perspektivische Darstellung einer weiteren Variante eines Abschirmblechs der Entladungslampe aus den Figuren 1 - 3. |

## Bevorzugte Ausführung der Erfindung

Zunächst wird zur Illustration des Aufbaus einer typischen dielektrisch behinderten Entladungslampe mit röhrenförmigem Entladungsgefäß verwiesen auf die bereits zuvor erwähnte EP 0 981 831. In diesem Dokument bereits gegebene Erläuterungen werden im weiteren nicht wiederholt. Stattdessen konzentriert sich die Schilderung der Ausführungsbeispiele auf die Unterschiede zu diesem Stand der Technik.

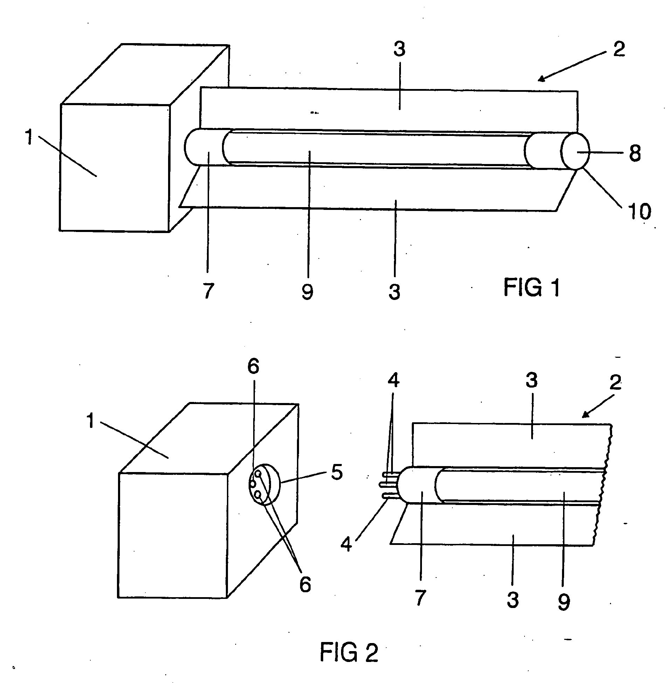

Figur 1 der vorliegenden Anmeldung zeigt ein erfindungsgemäßes Beleuchtungssystem mit einem elektronischem Vorschaltgerät 1, das hier als einfacher Quader dargestellt ist. Die Figur zeigt nur das Gehäuse des Vorschaltgeräts 1, das die im Übrigen an sich bekannten Schaltungsteile eines Vorschaltgeräts zum Betrieb einer dielektrisch behinderten Entladungslampe enthält. Dabei kann es sich insbesondere um einen Klasse-E-Konverter handeln.

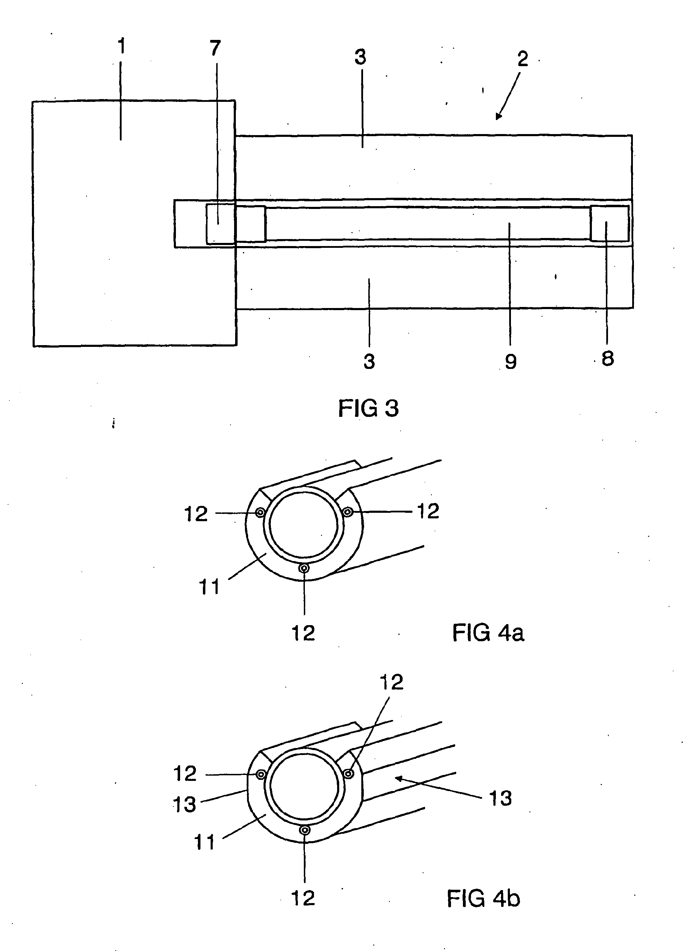

Die Figur zeigt, dass in den hinteren Bereich der in Figur 1 rechten Seite des Vorschaltgeräts 1 eine im Wesentlichen linienförmige dielektrisch behinderte Entladungslampe 2 mit zwei seitlich abstehenden Schirmflächen 3 eingesteckt ist. Figur 2 zeigt mit einem Ausschnitt des Vorschaltgeräts 1 und der Lampe 2 aus Figur 1 eine Situation, in der die Lampe 2 aus dem Vorschaltgerät 1 herausgezogen ist. Figur 3 zeigt eine Draufsicht auf die Situation aus Figur 1.

Man erkennt in Figur 2, dass ein Sockel 7 der röhrenförmigen Lampe 2 über die Schirmflächen 3 nach links hinausragt und dieser zylinderförmige hinausragende Sockel 7 drei weiterreichende axial verlaufende Elektrodenenden 4 aufweist. Ferner deutet Figur 2 an, dass das Vorschaltgerät 1 in seiner rechten Seitenfläche der im Übrigen quaderförmigen Gehäuseform eine dazu passende Steckbuchsenaufnahme 5 mit darin vorgesehenen weiblichen Steckverbindungselementen 6 für die erwähnten axialen Elektrodenenden 4 der Entladungslampe 2 aufweist.

Bei den axialen Elektrodenenden 4 handelt es sich um in den Figuren 1 - 3 linke Enden von rundstabförmigen Elektroden der Lampe 2, auf die anhand der Figuren 4 - 9 noch näher eingegangen wird. Diese Elektrodenenden werden gemäß Figur 2 zusammen mit dem über die Schirmflächen 3 hinausragenden Sockel 7 der Entladungslampe 2 in die beschriebenen Steckbuchse 5 mit den Steckverbindungselementen 6 eingesteckt. Dadurch ist, wie die Figuren 1 und 3 zeigen, die Lampe 2 nicht nur elektrisch an dem Vorschaltgerät 1 angeschlossen, sondern darüber hinaus auch fest an ihm montiert. Das Vorschaltgerät 1 dient also als Lampenhalterung. Ein flexibles Kabel zwischen Lampe 2 und Vorschaltgerät 1 kann daher entfallen.

Bei dem über die Schirmflächen 3 hinausreichenden Teil der Lampe 2 handelt es sich um einen Kunststoffsockel 7, der zusammen mit einem in den Figuren 1 und 3 erkennbaren zweiten Sockel 8 ein röhrenförmiges Glasentladungsgefäß 9 in einem die Schirmflächen 3 aufweisenden und im Folgenden noch näher beschriebenen Abschirmblech 10 hält. In den Figuren 2 und 3 ist das Abschirmblech 10 mit den Schirmflächen 3 elektrisch leitend mit dem metallischen Gehäuse des Vorschaltgeräts 1 verbunden. Dies kann beispielsweise durch einen in den Figuren 1 und 2 nicht dargestellten kleinen Stift geschehen, der am Außenumfang des Sockels 7 anliegt und mit diesem in die Steckbuchse 5 eingesteckt wird. Das Abschirmblech 10 ist gegenüber den Elektroden mit den Enden 4 über eine hier nicht dargestellte, aber in Figur 4 eingezeichnete Isolationslage isoliert. Hierbei handelt es sich um eine Kunststoffschicht. Diese Kunststoffisolierung liegt in dem in den Figuren 1 - 3 sichtbaren Teil des Entladungsgefäßes 9 zwischen den Schirmflächen 3, nämlich der Apertur zur Lichtabstrahlung, nicht vor. Das Abschirmblech 10 bildet mit den Sockeln 7 und 8 eine Manschette.

In Figur 4a ist das Abschirmblech 10 mit den Schirmflächen 3 der einfachen Darstellung halber weggelassen. Figur 4a zeigt eine Variante der erwähnten Kunststoffisolierung, in Form eines über die Lampenlänge laufenden Sockels 11 und im Übrigen Elektrodenenden 12, die zum einen nicht über den Sockel 11 hinausreichen, und die zum anderen Röhrenform haben. Hierbei handelt es sich um weibliche Steckverbindungselemente an den Elektrodenenden im Gegensatz zu den männlichen Steckverbindungselementen in Figur 2. Dementsprechend weist ein nicht dargestelltes komplementäres Vorschaltgerät männliche Steckverbindungselemente in einer Steckbuchse vergleichbar der Steckbuchse 5 aus Figur 2 auf. Die Elektroden sind in passende Ausnehmungen des Sockels 11 eingelegt und werden von ihm formschlüssig an dem Entladungsgefäß gehalten. Der Sockel 11 läuft über die Lampenlänge und geht in den Sockel (8 in den Figuren 1 und 3) an dem entgegengesetzten Lampenende über. Er wird durch das Abschirmblech 10 gegenüber dem Entladungsgefäß 9 unter Vorspannung gehalten und hält ohne weitere Maßnahmen daran. Das Entladungsgefäß 9 ist also eine einfache gasgefüllte Röhre mit inneren Leuchtstoff- und Reflexionsschichten.

Da hier die Isolationsschicht zwischen den Elektroden und dem Abschirmblech 10 gleichzeitig als Sockel entsprechend dem Sockel 7 aus Figur 2 ausgebildet ist, greift der Sockel also nicht um den gesamten Umfang des Entladungsgefäßendes herum.

In beiden Fällen, der Ausführungsform aus den Figuren 1 - 3 und der aus Figur 4a, besteht eine kraft- und formschlüssige Anlage des Abschirmblechs 10 um den Sockel und die Isolierung und gewährleistet damit eine Montageverbindung.

Figur 4b zeigt eine Variante zu Figur 4a, in dem dort zusätzliche Abflachungen 13 an den seitlichen Bereichen des Sockels 11 vorgesehen sind. Diese Abflachungen 13 sind in komplementärer Form an einem hier nicht zeichnerisch dargestellten Abschirmblech 10 entsprechend den Figuren 1 - 3 vorgesehen, so dass dadurch bereits eine korrekte Ausrichtung der Apertur auf die Schirmflächen 3 erfolgen kann.

Der Sockel 7 aus Figur 2 kann auch so ausgestaltet sein, dass er ausschließlich an den Enden des Entladungsgefäßes 9 eine entsprechende Abstandsjustage zu dem Abschirmblech 10 vorgibt und die Isolierung im axialen Zwischenbereich nur locker eingelegt ist oder im Fall beispielsweise innenliegender Elektroden ganz weggelassen ist.

Die in den Figuren 1 - 3 dargestellte Steckverbindung zwischen der Entladungslampe 2 und dem Vorschaltgerät 1 ist bei der Erfindung natürlich nicht obligatorisch. Als Steckverbindungselemente ausgebildete Elektrodenenden können auch ohne dieses Merkmal sinnvoll sein, beispielsweise wenn statt der Steckbuchse 5 des Vorschaltgeräts 1 ein entsprechender weiblicher Steckverbindungskopf eines Verbindungskabels vorgesehen ist, der zu den Elektrodenenden und optional auch ähnlich wie die Buchse 5 zu dem Sockel 7 bzw. dem Entladungsgefäß 9 passt.

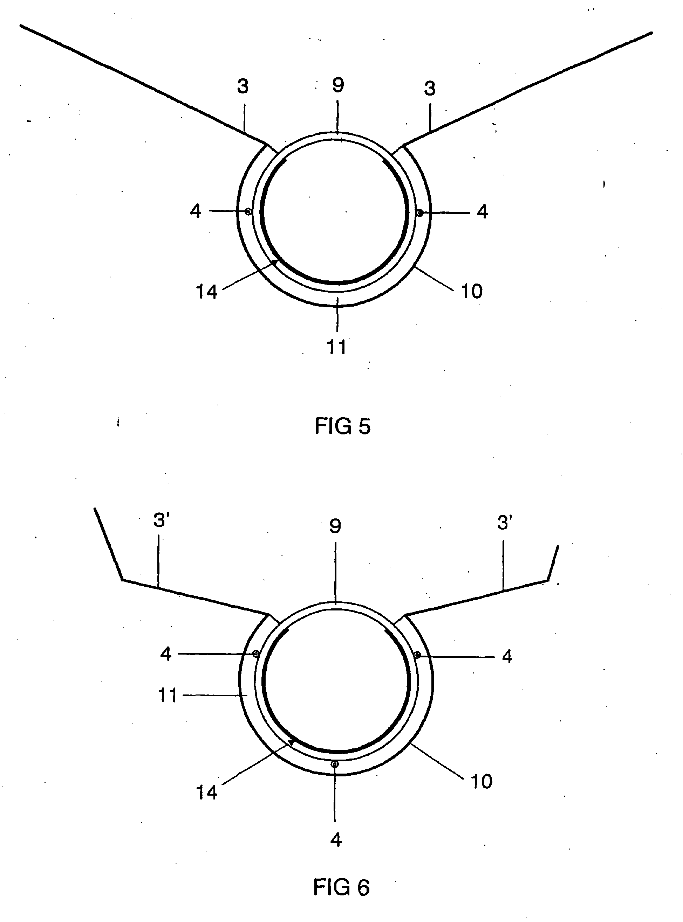

Die Figuren 5 - 9 zeigen einige Varianten zu den Entladungslampen gemäß den Figuren 1 - 4b. In Figur 5 sind statt wie in Figur 2 drei Elektroden (bzw. Elektrodenenden) 4 hier nur zwei Elektroden 4 vorgesehen. Beide Varianten sind möglich. Gelegentlich werden drei Elektroden gewählt, um eine bessere Lichtausbeute zu erzielen. Für die vorliegende Erfindung sind diese Unterschiede nicht von besonderem Belang. Ferner ist der Öffnungswinkel zwischen den Schirmflächen 3, also den flügelartigen Enden der Manschette 10 hier etwas kleiner gewählt. Dieser Öffnungswinkel ist jedoch immer noch so groß, dass er den tatsächlichen Lichtaustritt aus der Apertur im oberen Bereich des in Figur 5 dargestellten Schnitts nicht merklich behindert. Dennoch dienen diese Schirmflächen 3 Verbesserung der elektromagnetischen Abschirmung in seitlicher Richtung durch aus der Apertur austretende Streufelder. Figur 5 verdeutlicht die Apertur, indem dort eine Leuchtstoffschicht 14 eingezeichnet ist, die im Bereich der Apertur unterbrochen ist.

Figur 6 zeigt im Unterschied zu Figur 5 wieder drei Elektroden 4, der wesentliche Unterschied besteht jedoch darin, dass die Schirmflächen 3' aus Figur 6 hier um nach innen gewinkelte Teile ergänzt sind und- damit einen noch etwas engeren Öffnungswinkel begrenzen. Dieser ist bezogen auf den Kreismittelpunkt des Entladungsgefäßes noch immer deutlich größer als der Öffnungswinkel der Apertur. Da jedoch auch die Randbereiche der Leuchtstoffschicht 14 Licht abstrahlen, werden die äußersten Bereiche der Lichtabstrahlung bereits abgeblendet. Die Abschirmwirkung ist aber dementsprechend verbessert.

Die gewinkelte Form der Schirmflächen 3' kann dabei auf bauliche Gegebenheiten in der Umgebung Rücksicht nehmen, etwa wenn das Beleuchtungssystem (im Sinn der Figur 1) in einer Umgebung mit vorgegebenen räumlichen Verhältnissen angebracht werden soll, oder wenn eine solche Formgebung zu Montagezwecken vorteilhaft erscheint. Figur 1 hat bereits verdeutlicht, dass das Abschirmblech 10 nicht nur zur Halterung der Elektroden an dem Entladungsgefäß 9 dient, sondern auch die Montage der gesamten Entladungslampe 2 an dem Vorschaltgerät 1 stabilisiert. Bei Bedarf können die Schirmflächen 3 auch eigens montiert werden, etwa an dem Vorschaltgerät 1 angeklemmt, gesteckt oder geschraubt werden. Im Übrigen können sie auch gegenüber anderen Bauteilen als dem Vorschaltgerätgehäuse eine Montagefunktion haben.

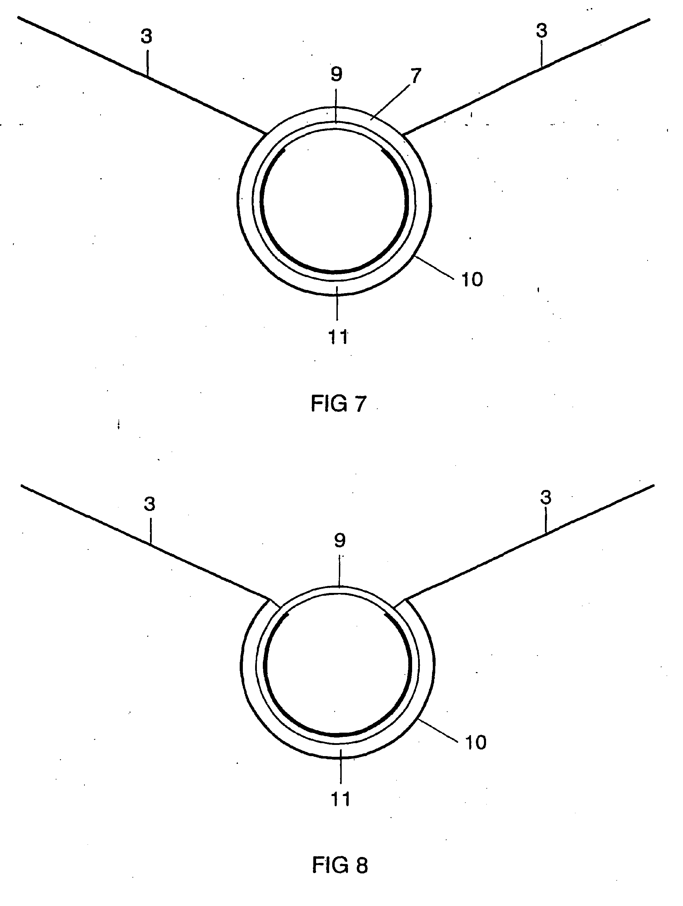

Figur 7 zeigt eine weitere Variante zu Figur 5 mit einem wiederum verengten Öffnungswinkel der Schirmflächen 3, hier jedoch mit geraden Schirmflächen 3. In diesem Fall läuft der Sockel 7 entsprechend Figur 2 um den gesamten Umfang des Entladungsgefäßes 9 und spart nicht, wie in Figur 4, die Apertur aus. Da der Sockel 7 allerdings nur am äußersten Rand angebracht ist, stört dies die Lichtabstrahlung nicht oder kaum.

Figur 8 unterscheidet sich gerade durch dieses letztgenannte Merkmal von Figur 7. Hier ist wiederum der Apertur ausgespart. Es handelt es sich also um einen Sockel 11 entsprechend Figur 4.

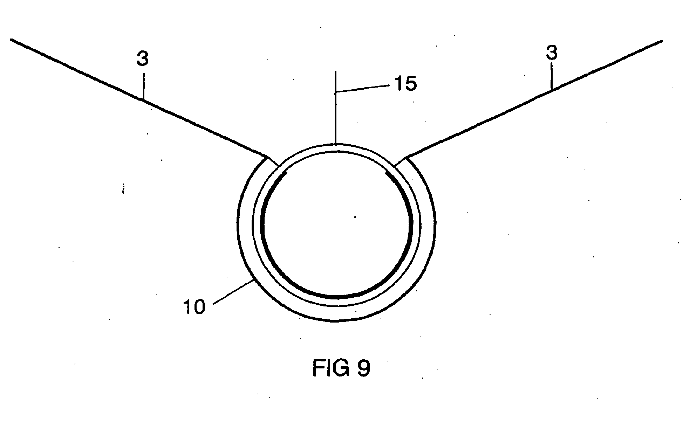

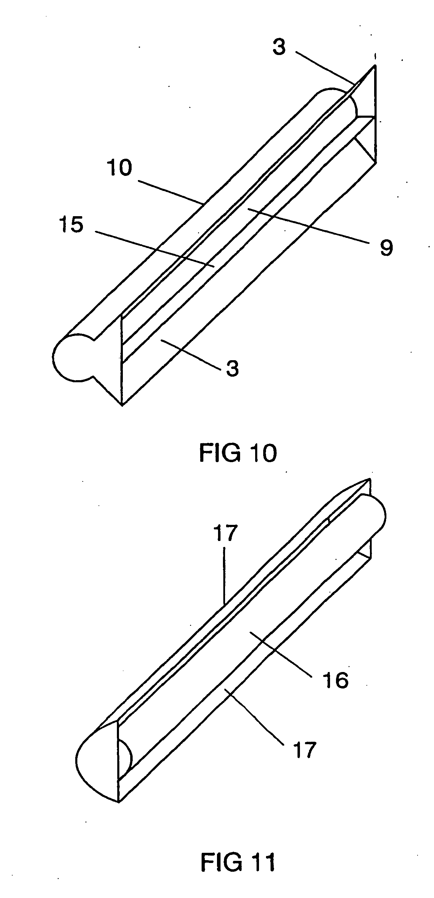

Figur 9 unterscheidet sich von Figur 8 durch ein zusätzliches Abschirmungsteil 15 in dem Öffnungswinkel sowohl der Schirmflächen 3 als auch der Apertur. Dieses ist im dargestellten Querschnitt radial und im Übrigen flächig-ausgestaltet und in der perspektivischen Ansicht in Figur 10 besser erkennbar. Es reduziert die Lichtabstrahlung durch die Apertur geringfügig, verbessert allerdings die elektromagnetische Abschirmung in Lichtabstrahlungsrichtung zusätzlich. Ein solches Teil 15 kann eine kostengünstige Alternative oder auch zusätzliche Maßnahme zu einer transparenten leitfähigen Beschichtung der Apertur sein, wie sie in der bereits zitierten EP-Schrift dargestellt ist. Der Übersichtlichkeit halber sind die Einzelheiten der Steckverbindung in Figur 10 weggelassen.

Figur 11 zeigt in einer Figur 10. ähnelnden Darstellung eine Variante zu der Gestaltung des Abschirmblechs 10. Hier besteht das Abschirmblech 10 mit den Schirmflächen im Schnitt betrachtet im Grunde aus zwei konzentrischen Halbkreisen 16 und 17 mit wesentlich unterschiedlichem Durchmesser um den Kreismittelpunkt des Schnitts durch das Entladungsgefäß 9. Die Halbkreise 16, 17 sind mit ihren Öffnungen einander zugewandt. Im Unterschied zu den bisherigen Varianten zeigt dabei auch der kleinere der Halbkreise 16 einen deutlich größeren Abstand von dem Entladungsgefäß 9, das hier nicht eingezeichnet ist. Dadurch dient schon der kleinere Halbkreis 16 als Reflektor, reflektiert das von der Apertur in ihn hinein (d. h. in Figur 11 nach rechts) abgestrahlte Licht in den größeren Halbkreis 17, der das Licht wiederum aus der Manschette heraus reflektiert. Diese Variante bietet eine deutlich schlechtere Lichtausbeute als die bisherigen Beispiele, zeigt jedoch eine erheblich bessere EMV-Abschirmung.

**Patentansprüche**

**1.** Dielektrisch behinderte Entladungslampe
mit einem röhrenförmig langgestreckten Entladungsgefäß
und mit einer leitfähigen metallischen Abschirmung, die das Entladungsgefäß teilweise umgreift und dabei in einer Schnittebene senkrecht zur Längserstreckungsrichtung des Entladungsgefäßes einen Öffnungswinkel zur Lichtabstrahlung freilässt,
**gekennzeichnet durch** zumindest eine sich längs des Entladungsgefäßes erstreckende und den Öffnungswinkel begrenzende Schirmfläche der Abschirmung, die von dem Entladungsgefäß an ihrem äußersten Ende um eine Strecke entfernt ist, die mindestens so groß wie der halbe mittlere Durchmesser des Entladungsgefäßes quer zur Längserstreckung ist.

2. Entladungslampe nach Anspruch 1, bei der die Abschirmung eine das Entladungsgefäß umschließende Manschette bildet, die das Entladungsgefäß um mehr als die Hälfte seines Umfangs umgreift.

3. Entladungslampe nach Anspruch 2, bei der die Abschirmung das Entladungsgefäß zu einem Teil seines Umfangs in einem im Verhältnis zu dem mittleren halben Durchmesser des Entladungsgefäßes quer zur Längserstreckung kleinen Abstand umgibt und von diesem Bereich der Abschirmung ausgehend zumindest ein flächiger Abschirmungsbereich absteht, der die mit ihrem äußersten Ende entfernte Schirmfläche bildet.

4. Entladungslampe nach einem der vorstehenden Ansprüche, bei dem der Lichtabstrahlungsöffnungswinkel der Abschirmung größer als ein Öffnungswinkel einer Lichtabstrahlungsapertur der Entladungslampe selbst ist, wobei beide Öffnungswinkel auf den Mittelpunkt des Entladungsgefäßes im Querschnitt zur Längsrichtung bezogen sind.

5. Entladungslampe nach einem der vorstehenden Ansprüche, bei der innerhalb des Lichtabstrahlungsöffnungswinkels der Abschirmung weitere Abschirmungsteile vorgesehen sind.

6. Entladungslampe nach Anspruch 5, bei der die innerhalb des Öffnungswinkels vorgesehenen Abschirmungsteile flächig ausgebildet und im Wesentlich radial in Bezug auf das Entladungsgefäß orientiert sind.

7. Entladungslampe nach Anspruch 3 auch in Verbindung mit einem der Ansprüche 4 - 6, bei der für eine Dicke $d_D$ der Isolierung zwischen der Abschirmung und der Elektrode, eine Dielektrizitätszahl $\varepsilon_D$ derselben, eine Dicke $d_B$ einer dielektrischen Barriere zwischen der Elektrode und einem Entladungsmedium und eine Dielektrizitätszahl $\varepsilon_B$ derselben insgesamt die Beziehung gilt:

$$d_D\,/\,\varepsilon_D \geq F \times d_B\,/\,\varepsilon_B,$$

wobei der Faktor F größer 1,5 ist.

8. Entladungslampe nach Anspruch 7, bei der die Abschirmung an zumindest einem endseitigen Sockel der Lampe umlaufend anliegt.

9. Entladungslampe nach Anspruch 8, bei der der Sockel und die Abschirmung im Querschnitt zu der Längserstreckung der Lampe Abflachungen aufweisen, die bei der Montage der Abschirmung die richtige Orientierung im Drehsinn um die Längserstreckung der Lampe vorgeben.

10. Entladungslampe nach einem der vorstehenden Ansprüche mit zumindest einer Elektrode, die entlang der Längserstreckung des Entladungsgefäßes an der Außenseite des Entladungsgefäßes angebracht ist, wobei die Elektrode an dem Entladungsgefäß durch Formschluss mit der die Elektrode umgreifenden Abschirmung angebracht ist.

11. Entladungslampe nach einem der vorstehenden Ansprüche mit zumindest zwei stäbförmigen Elektroden, die an der Außenseite des Entladungsgefäßes angebracht sind, wobei die Elektroden an einem Ende als Steckverbindungselement ausgebildet sind.

12. Beleuchtungssystem mit einer Entladungslampe nach einem der vorstehenden Ansprüche und einem elektronischen Vorschaltgerät zum Betrieb der Lampe, an dem die Entladungslampe elektrisch angeschlossen ist, wobei die elektrische Verbindung zwischen dem Vorschaltgerät und der Entladungslampe abgeschirmt ist.

13. Beleuchtungssystem nach Anspruch 12, bei dem die Abschirmung der Entladungslampe mit dem Gehäuse des Vorschaltgeräts direkt, elektrisch leitend und mechanisch fest verbunden ist und die Entladungslampe relativ zu dem Gehäuse hält.

14. Beleuchtungssystem nach Anspruch 12 oder 13, bei dem die Entladungslampe an einem Ende des Entladungsgefäßes angebrachte Kontakte zum elektrischen Anschluss der Lampe aufweist, wobei mit einem Gehäuse des Vorschaltgeräts ein Steckverbindungselement fest verbunden ist, das so ausgelegt ist, dass die Lampe mit dem die Kontakte aufweisenden Ende als komplementäres Steckverbindungselement durch Zusammenstecken mit dem Steckverbindungselement des Gehäuses an das Vorschaltgerät angeschlossen werden kann.

15. Verwendung einer Entladungslampe nach einem der Ansprüche 1 - 11 oder eines Beleuchtungssystems nach Anspruch 12, 13 oder 14 als UV-Strahler zur Beleuchtung eines Katalysators.

16. Verwendung nach Anspruch 15, bei der der Katalysator zur Luftreinigung in einem Fahrzeug dient.

**Claims**

1. Discharge lamp having a discharge vessel which is elongate in the form of a tube and having a conductive metallic shield which partially surrounds the discharge vessel and in the process leaves an angle of opening free for light radiation purposes on a sectional plane perpendicular to the direction of longitudinal extent, **characterized by** at least one shielding face, extending along the discharge vessel, and, limiting the angle of opening, of the shield being remote from the discharge vessel at its outermost end by a distance which is at least as great as half the average diameter of the discharge vessel transverse to the longitudinal extent.

2. Discharge lamp according to Claim 1, in which the shield forms a sleeve which surrounds the discharge vessel and engages around the discharge vessel over more than half its circumference.

3. Discharge lamp according to Claim 2, in which the shield surrounds the discharge vessel over part of its circumference at a smaller distance than half the average diameter of the discharge vessel, transverse to the longitudinal extent, and, starting from this region of the shield, at least one flat shielding region protrudes which forms the shielding face remote with its outermost end.

4. Discharge lamp according to one of the preceding claims, in which the light radiation angle of opening of the shield is greater than an angle of opening of a light radiation aperture of the discharge lamp itself, the two angles of opening being based on the central point of the discharge vessel in cross section with respect to the longitudinal direction.

5. Discharge lamp according to one of the preceding claims, in which further shielding parts are provided within the light radiation angle of opening of the shield.

6. Discharge lamp according to Claim 5, in which the shielding parts provided within the angle of opening are flat and are oriented substantially radially with respect to the discharge vessel.

7. Discharge lamp according to Claim 3, also in connection with one of Claims 4 - 6, in which the following relationship applies overall to a thickness $d_D$ of the insulation between the shield and the electrode, a dielectric constant $\varepsilon_D$ thereof, a thickness $d_B$ of a dielectric barrier between the electrode and a discharge medium and a dielectric constant $\varepsilon_D$ thereof:

$$d_D/\varepsilon_D \geq F \times d_B/\varepsilon_B,$$

where the factor F is greater than 1.5.

8. Discharge lamp according to Claim 7, in which the shield bears circumferentially against at least one end-side base of the lamp.

9. Discharge lamp according to Claim 8, in which the base and the shield have flattened sections in cross section with respect to the longitudinal extent of the lamp, said flattened sections providing the correct orientation of the lamp in the rotational sense about the longitudinal extent of the lamp when the shield is mounted.

10. Discharge lamp according to one of the preceding claims having at least one electrode which is fitted to the outside of the discharge vessel along the longitudinal extent of the discharge vessel, the electrode being fitted to the discharge vessel by means of an interlocking connection with the shield surrounding the electrode.

11. Discharge lamp according to one of the preceding claims having at least two rod-shaped electrodes which are fitted to the outside of the discharge vessel, the electrodes being in the form of a plug connection element at one end.

12. Illumination system having the discharge lamp according to one of the preceding claims and an electronic ballast for the purpose of operating the lamp to which the discharge lamp is electrically connected, the electrical connection between the ballast and the discharge lamp being shielded.

13. Illumination system according to Claim 12, in which the shield of the discharge lamp is directly, electrically conductively and mechanically fixedly connected to the housing of the ballast and holds the discharge lamp in relation to the housing.

14. Illumination system according to Claim 12 or 13, in which the discharge lamp has contacts, which are fitted at one end of the discharge vessel, for electrically connecting the lamp, a plug connection element being fixedly connected to a housing of the ballast, said plug connection element being designed such that the lamp can be connected to the ballast with the end having the contacts as the complementary plug connection element by being plugged together with the plug connection element of the housing.

15. Use of the discharge lamp according to one of Claims 1-11 or the illumination system according to Claim 12, 13 or 14 as a UV radiator for the purpose of illuminating a catalyst.

16. Use according to Claim 15, in which the catalyst is used for air purification purposes in a vehicle.

**Revendications**

1. Lampe à décharge rendue incomplète par voie diélectrique
comprenant une enceinte de décharge tubulaire, s'étendant en longueur
et comprenant un écran métallique, conducteur, qui entoure en partie l'enceinte de décharge et qui dégage ainsi, dans un plan de coupe perpendiculairement à la direction d'étendue en longueur de l'enceinte de décharge, un angle d'ouverture pour l'émission de lumière,
**caractérisée par** au moins une surface de l'écran, qui s'étend le long de l'enceinte de décharge, puis délimite l'angle d'ouverture et qui est éloignée de l'enceinte de décharge à son extrémité la plus à l'extérieur d'une étendue qui est au moins aussi grande que la moitié du diamètre moyen de l'enceinte de décharge transversalement à l'étendue longitudinale.

2. Lampe à décharge suivant la revendication 1, dans laquelle l'écran forme une manchette entourant l'enceinte de décharge et l'enserrant sur plus de la moitié de son pourtour.

3. Lampe à décharge suivant la revendication 2, dans laquelle l'écran entoure l'enceinte de décharge sur une partie de son pourtour à une distance petite par rapport à la moitié du diamètre moyen de l'enceinte de décharge transversalement à l'étendue en longueur et au moins une partie plate de l'écran qui forme, par son extrémité la plus extérieure, la surface d'écran éloignée, s'écarte en partant de cette partie de l'écran.

4. Lampe à décharge suivant l'une des revendications précédentes, dans laquelle l'angle d'ouverture d'émission de la lumière de l'écran est plus grand qu'un angle d'ouverture d'une ouverture d'émission de lumière de la lampe à décharge soi-même, les deux angles d'ouverture étant rapportés au centre de l'enceinte de décharge dans la section transversale par rapport à la direction longitudinale.

5. Lampe à décharge suivant l'une des revendications précédentes, dans laquelle il est prévu d'autres parties d'écran à l'intérieur de l'angle d'ouverture d'émission de la lumière de l'écran.

6. Lampe à décharge suivant la revendication 5, dans laquelle les parties d'écran prévues à l'intérieur de l'angle d'ouverture sont plates et sont orientées sensiblement radialement par rapport à l'enceinte de décharge.

7. Lampe à décharge suivant la revendication 3 également en liaison avec l'une des revendications 4 à 6, dans laquelle, pour une épaisseur $d_D$ de l'isolant entre l'écran et l'électrode, une constante $\varepsilon_D$ diélectrique de celle-ci, une épaisseur $d_B$ d'une barrière diélectrique entre l'électrode et un milieu de décharge et une constante $\varepsilon_B$ diélectrique de celui-ci satisfont dans l'ensemble à la relation :

$$d_D/\varepsilon_D \ \geq \ F \ \textbf{X} \ d_B/\varepsilon_B$$

dans laquelle le facteur F est plus grand que 1,5.

8. Lampe à décharge suivant la revendication 7, dans laquelle l'écran s'applique tout autour à au moins un culot de la lampe du côté de l'extrémité.

9. Lampe à décharge suivant la revendication 8, dans laquelle le culot et l'écran ont, en section transversale par rapport à l'étendue longitudinale de la lampe, des méplats qui prescrivent, lors du montage de l'écran, la bonne orientation dans le sens de rotation autour de l'étendue longitudinale de la lampe.

10. Lampe à décharge suivant l'une des revendications précédentes, comprenant au moins une électrode qui est mise le long de l'étendue longitudinale de l'enceinte de décharge sur la face extérieure de l'enceinte de décharge, l'électrode étant mise sur l'enceinte de décharge par complémentarité de forme avec l'écran enserrant l'électrode.

11. Lampe à décharge suivant l'une des revendications précédentes, comprenant au moins deux électrodes en forme de barreau qui sont mises sur la face extérieure de l'enceinte de décharge, les électrodes étant formées à une extrémité en élément de liaison par enfichage.

12. Système d'éclairage ayant une lampe à décharge suivant l'une des revendications précédentes et un ballast électronique pour faire fonctionner la lampe, auquel la lampe à décharge est raccordée électriquement, la liaison électrique entre le ballast et la lampe à décharge étant protégée.

13. système d'éclairage suivant la revendication 12, dans lequel l'écran de la lampe à décharge est relié directement d'une manière conductrice d'électricité et solide mécaniquement au boîtier du ballast et retient la lampe à décharge par rapport au boîtier.

14. système d'éclairage suivant la revendication 12 ou 13, dans lequel la lampe à décharge a, pour le raccordement électrique des contacts mis à une extrémité de l'enceinte de décharge, un élément de liaison par enfichage étant assemblé fixement à un boîtier du ballast et étant conçu de façon à ce que la lampe puisse être raccordée à l'extrémité ayant les contacts en élément de liaison par enfichage complémentaire par enfichage avec l'élément de liaison par enfichage du boîtier sur le ballast.

15. Utilisation d'une lampe à décharge suivant l'une des revendications 1 à 11 pour un système d'éclairage suivant la revendication 12, 13 ou 14 comme source d'UV pour éclairer un catalyseur.

16. Utilisation suivant la revendication 15, dans laquelle le catalyseur sert à la purification de l'air d'un véhicule automobile.

FIG 1

FIG 2

FIG 3

FIG 4a

FIG 4b

FIG 5

FIG 6

FIG 7

FIG 8

FIG 9

**FIG 10**

**FIG 11**

**EP 1 643 538 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0981831 A